# EUROPÄISCHE PATENTSCHRIFT

(11) **EP 1 461 330 B1**
(45) Veröffentlichungstag und Bekanntmachung des Hinweises auf die Patenterteilung: **12.03.2008**
(21) Anmeldenummer: 02796714.0
(22) Anmeldetag: 20.12.2002
(51) Int. Cl.: C07D 311/92, C07D 405/04, G03C 1/73, G02B 5/23

(54) **3H-NAPHTHO[2,1-b]-PYRAN-DERIVATE SOWIE DEREN VERWENDUNG**
3H-NAPHTHO[2,1-b]-PYRANE DERIVATIVES AND THEIR USE
DERIVES DE 3H-NAPHTHO[2,1-b]-PYRANNE ET LEUR UTILISATION

(30) Priorität: 03.01.2002 DE 10200040
(43) Veröffentlichungstag der Anmeldung: 29.09.2004
(73) Patentinhaber: Rodenstock GmbH, 80469 München (DE)
(72) Erfinder: MANN, Claudia, 80634 München (DE); MELZIG, Manfred, 82234 Wessling (DE); WEIGAND, Udo, 80638 München (DE)
(74) Vertreter: Müller-Boré & Partner Patentanwälte
(86) Internationale Anmeldenummer: PCT/EP2002/014688
(87) Internationale Veröffentlichungsnummer: WO 2003/055872

(56) Entgegenhaltungen:
- EP-A- 0 945 451
- WO-A-01/12619
- WO-A-98/45281
- US-A- 5 238 981
- US-A- 5 990 305
- PATENT ABSTRACTS OF JAPAN vol. 1996, no. 10, 31. Oktober 1996 (1996-10-31) -& JP 08 157467 A (TOKUYAMA CORP), 18. Juni 1996 (1996-06-18)
- PATENT ABSTRACTS OF JAPAN vol. 1996, no. 11, 29. November 1996 (1996-11-29) -& JP 08 176139 A (TOKUYAMA CORP), 9. Juli 1996 (1996-07-09)

## Beschreibung

Die vorliegende Erfindung betrifft spezifische photochrome 3H-Naphtho[2,1-b]-pyran-Derivate sowie deren Verwendung in Kunststoffen aller Art, insbesondere für ophthalmische Zwecke. Insbesondere betrifft die vorliegende Erfindung von 3H-Naphtho[2,1-b]-pyranen abgeleitete photochrome Verbindungen, die in der offenen Form besonders langwellige Absorptionsmaxima aufweisen, wodurch bei Anwendung in phototropen Gläsern violette bis blaue Farbtöne erreicht werden können.

Es sind verschiedene Farbstoffklassen bekannt, die bei Bestrahlung mit Licht bestimmter Wellenlängen, insbesondere Sonnenstrahlen, reversibel ihre Farbe wechseln. Dies rührt daher, daß diese Farbstoffmoleküle durch Energiezufuhr in Form von Licht in einen angeregten farbigen Zustand übergehen, den sie bei Unterbrechung der Energiezufuhr wieder verlassen, wodurch sie in ihren farblosen oder zumindest kaum gefärbten Normalzustand zurückkehren. Zu diesen photochromen Farbstoffen gehören beispielsweise die Naphthopyrane, die im Stand der Technik mit verschiedenen Substituenten bereits beschrieben wurden.

Pyrane, speziell Naphthopyrane und von diesen abgeleitete größere Ringsysteme, sind photochrome Verbindungen, die bis heute Gegenstand intensiver Untersuchungen sind. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen.

Die im Stand der Technik bekannten Farbstoffe weisen dabei zum einen häufig eine nicht ausreichend langwellige Absorption im angeregten wie im nicht angeregten Zustand auf. Dies führt auch bei Kombinationen mit weiteren photochromen Farbstoffen zu Problemen. Zum anderen liegt häufig auch eine zu hohe Temperaturempfindlichkeit bezüglich der Eindunkelung vor, wobei gleichzeitig eine zu langsame Aufhellung eintritt. Darüberhinaus weisen die im Stand der Technik verfügbaren Farbstoffe häufig eine ungenügende Lebensdauer auf. Infolgedessen zeigen derartige Sonnenschutzgläser eine unzureichende Haltbarkeit. Letzteres macht sich in schnell nachlassender Leistung und/oder starker Vergilbung bemerkbar.

Von 2-Naphtholen abgeleitete 3H-Naphthopyrane und ihre davon durch Annellierung abgeleiteten höheren analogen Derivate sind eine Gruppe von photochromen Farbstoffen, deren längstwelliges Absorptionsmaximum der angeregten Form vorwiegend im Spektralbereich von 420 nm bis 500 nm liegt und somit einen gelben, orangen oder roten Farbeindruck vermitteln (siehe US-A-5,869,658 sowie US-A-6,022,495). Für neutraleindunkelnde phototrope Gläser werden jedoch leistungsstarke violette bis blaue photochrome Farbstoffe benötigt. Violette bis blaue photochrome Farbstoffe, die derzeit im Stand der Technik verfügbar sind, stammen üblicherweise aus der Klasse der Spiroxazine, Fulgide oder 2H-Naphtho[1,2-b]pyrane. Spiroxazin-Farbstoffe sind jedoch in der Regel hinsichtlich der Hochtemperaturleistung nachteilig, während Fulgid-Farbstoffe in der Lebensdauer und 2H-Naphtho[1,2-b]pyrane in der Aufhellgeschwindigkeit keine für die Anwendung in Sonnenschutzgläser vollständig zufriedenstellenden Eigenschaften aufweisen.

Die Einführung von elektronenschiebenden Substituenten an den Arylresten in o-Stellung zum Pyransauerstoff, wie beispielsweise in WO 98/45281 und WO 01/12619 und EP 0 945451 A1 beschrieben, führt zu rot bzw. rotviolett eindunkelnden 3H-Naphtho[2,1-b]pyranen. In WO 01/12619 werden Verbindungen of fenbart, deren eine geminale Arylgruppe eine para-aminosubstituierte Gruppe und deren andere Arylgruppe eine in meta- oder para-Stellung substituierte Alkoxy- oder Thioalkoxygruppe aufweist, wobei dieses Substitutionsmuster einen positiven Einfluß auf die Aufhellgeschwindigkeit bewirkt. In WO 98/45281 werden rote hyperchrome Verbindungen beschrieben, die zusätzlich eine Aminfunktion vorrangig in 6-Stellung der 3H-Naphtho[2,1-b]pyran-Einheit enthalten. Verbindungen mit nicht ausgeprägten basischen Aminogruppen werden in EP 0 945 451 A1 beschrieben, die im angeregten Zustand eine pinkfarbene bis violette Farbe zeigen sowie eine ansprechende Lebensdauerleistung aufweisen. Entsprechende Substitution in der 8-Stellung der 3H-Naphtho[2,1-b]pyran-Einheit bewirkt eine bathochrome Verschiebung des längstwelligen Absorptionsmaximums, vor allem durch die Einführung von Alkoxygruppen, wie in US-A-5,238,981 beschrieben. Außerdem sind auch Verbindungen mit Dialkylaminogruppen in 8-Stellung offenbart. Die Verwendung von Stickstoff-Heterocyclen als Substituenten in der 8-Stellung der 3H-Naphtho[2,1-b]pyran-Einheit wird in US-A-5,990,305 erwähnt, wodurch im Gegensatz zu offenkettigen Aminogruppen eine verbesserte Lebensdauer erzielt wird. Diese wird auch mit Substituenten erreicht, die sog. HALS-(hindered amine light stabilizer) Strukturelemente enthalten.

Somit liegt der vorliegenden Erfindung die Aufgabe zugrunde, neue photochrome Farbstoffe bereitzustellen, die verbesserte Eigenschaften gegenüber den im Stand der Technik beschriebenen Verbindungen aufweisen sollen. Die photochromen Verbindungen sollen sich gegenüber vergleichbaren Verbindungen aus dem Stand der Technik insbesondere durch eine im angeregten Zustand längerwellige Absorption bei gleichzeitig guten Kinetik- und Lebensdauereigenschaften, d.h. schnelle Aufhellungsgeschwindigkeit sowie gutes Verhalten im Lebensdauertest, auszeichnen.

Diese Aufgabe wird durch die in den Ansprüchen gekennzeichneten Gegenstände gelöst.

Insbesondere werden photochrome 3H-Naphtho[2,1-b]-pyran-Derivate mit der allgemeinen Formel (I) bereitgestellt: 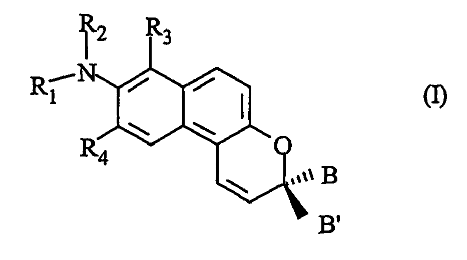 worin
die Reste R₁ und R₂ unabhängig voneinander einen Substituenten darstellen, ausgewählt aus
der Gruppe α, bestehend aus einem geradkettigen oder verzweigtkettigen (C₁-C₆)-Alkylrest, einem (C₃-C₇)-Cycloalkylrest und einem un-, mono- oder disubstituierten Phenyl-, Phenoxy-, Benzyl-, Benzyloxy-, Naphthyl- oder Naphthoxyrest, wobei die Substituenten aus geradkettigen oder verzweigtkettigen (C₁-C₆)-Aikylresten und (C₁-C₆)-Alkoxyresten ausgewählt sein können,
der Gruppe β, worin die Reste R₁ und R₂ zusammen mit dem Stickstoffatom einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁵ enthalten kann, wobei der Rest R⁵ aus einem geradkettigen oder verzweigtkettigen (C₁-C₆)-Alkylrest oder einem unsubstituierten oder mit geradkettigen oder verzweigtkettigen (C₁-C₆)-Alkylresten mono- oder disubstituierten Phenyl- oder Benzylrest ausgewählt sein kann, und wobei der Stickstoffheterocyclus mit einem oder zwei Benzolringen annelliert sein kann, oder
der Gruppe χ, worin die Reste R₁ und R₂ zusammen mit dem Stickstoffatom eine Azaadamantylgruppe bilden;
die Reste R₃ und R₄ unabhängig voneinander aus Wasserstoff, einem geradkettigen oder verzweigtkettigen (C₁-C₆)-Alkylrest, einem (C₃-C₇)-Cycloalkylrest oder einem (C₁-C₆)-Alkoxyrest ausgewählt sind, oder
die Reste R₁ und R₄ bzw. R₂ und R₃ unter Einschließen des Stickstoffatoms jeweils miteinander eine an den Benzolring des Naphthopyrangerüsts gebundene -R₂N-(CH₂)ₖ-X- bzw. -R₁N-(CH₂)ₖ-X- Einheit mit k = 1 oder 2 bilden, wobei X aus O, S, CH₂, C(CH₃)₂, C(C₆H₅)₂, N(CH₃) oder N(C₆H₅) ausgewählt ist und der Rest R₂ bzw. R₁ dann aus Methyl oder Phenyl ausgewählt ist, und wobei an diese -R₂N-(CH₂)ₖ-X- bzw. -R₁N-(CH₂)ₖ-X- Einheit ein Benzolring annelliert sein kann, oder
die Reste NR₁R₂, R₃ und R₄ zusammen mit dem Benzolring des Naphthopyrangerüsts, an den sie gebunden sind, eine Julolidinyl-Einheit bilden,

B ausgewählt ist:
aus einem an den Pyranring über die 3-Position gebundenen Julolidinylrest, einem in ortho- oder para-Stellung mono- oder disubstituierten Phenyl- oder Naphthylrest, wobei der oder die Substituenten eine -NR₆R₇ Gruppe ist bzw. sind, wobei die Reste R₆ und R₇ unabhängig voneinander aus einem (C₃-C₇)-Cycloalkylrest oder einem gegebenenfalls mit einem oder mehreren geradkettigen oder verzweigtkettigen (C₁-C₆)-Alkylresten oder (C₁-C₆)-Alkoxyresten substituierten Phenylrest oder Benzylrest ausgewählt sind, oder die Reste R₆ und R₇ zusammen mit dem Stickstoffatom der -NR₆R₇ Gruppe eine Azaadamantylgruppe oder einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁵ enthalten kann und gegebenenfalls mit einem oder zwei Benzolringen annelliert sein kann, oder zwei direkt benachbarte Substituenten an dem Phenyl- oder Naphthylrest eine an den aromatischen Ring gebundene -R₈N-(CH₂)ₖ-X- Gruppe mit k = 1 oder 2 bilden, wobei X aus O, S, CH₂, C(CH₃)₂, C(C₆H₅)₂, N(CH₃) oder N(C₆H₅) ausgewählt ist und der Rest R₈ dann aus Methyl oder Phenyl ausgewählt ist, mit der Maßgabe, daß die -R₈N- Einheit in ortho- oder para-Stellung gebunden ist,
B' aus einem unsubstituierten oder in ortho- oder para-Stellung mono-, di- oder trisubstituierten Phenyl- oder Naphthylrest ausgewählt ist, wobei die Substituenten aus einem geradkettigen oder verzweigtkettigen (C₁-C₆)-Alkylrest, einem (C₃-C₇)-Cycloalkylrest, einem (C₁-C₆)-Alkoxyrest, Fluor, Chlor oder Brom ausgewählt sind, wobei die Phenyl- bzw. Naphthylreste der Einheiten B und B' unabhängig voneinander direkt, über eine Ethylengruppe oder eine Ethindiylgruppe (d.h. Acetylengruppe) an der 3,3'-Position des 3H-Naphtho[2,1-b]-pyransystems gebunden sein können.

Durch Einführung mindestens zweier Aminfunktionen, sowohl in der 8-Position am Naphthopyran-Grundgerüst als auch an einem der geminalen Arylreste in 3-Stellung des Naphthopyran-Grundgerüsts, lassen sich die photochromen Eigenschaften von 3H-Naphtho[2,1-b]-pyranen deutlich steigern. Gegenüber entsprechenden Verbindungen aus dem Stand der Technik werden erstmals 3H-Naphtho[2,1-b]pyrane bereitgestellt, die in der angeregten Form deutlich länger wellig absorbieren. Dadurch lassen sich violette bis blaue photochrome Farbstoffe in nur wenigen Reaktionsschritten herstellen. Darüber hinaus zeigen die erfindungsgemäßen photochromen 3H-Naphtho[2,1-b]-pyrane gute Lebensdauer und schnelle Aufhellgeschwindigkeiten bei gleichzeitig guter Eindunkelungsleistung.

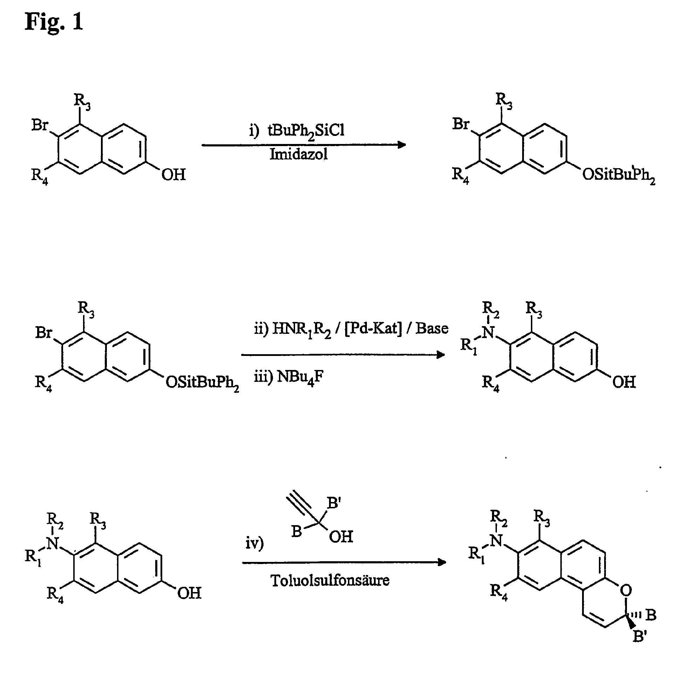

Fig. 1 zeigt exemplarisch einen Syntheseweg zur Herstellung beispielhafter erfindungsgemäßer photochromer Verbindungen.

In einer bevorzugten Ausführungsform der vorliegenden Erfindung werden die Reste R₁ und R₂ gemäß der vorstehenden Formel (I) aus einem unsubstituierten, mono- oder disubstituierten Phenyl-, Phenoxy-, Benzyl-, Benzyloxy-, Naphthyl- oder Naphthoxyrest ausgewählt oder bilden zusammen mit dem Stickstoffatom einen 3- bis 10-gliedrigen Stickstoffheterocyclus, insbesondere eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine (N'-(C₁-C₆-Alkyl)piperazingruppe, eine Pyrrolidingruppe, eine Imidazolidingruppe, eine Pyrazolidingruppe, eine Aziridingruppe, eine Azetidingruppe, eine Indolingruppe, eine Carbazolgruppe, eine Phenothiazingruppe, eine Phenazingruppe, eine Phenoxazingruppe, eine Tetrahydrochinolingruppe und eine Tetrahydroisochinolingruppe. Mehr bevorzugt steht die NR₁R₂-Gruppe in der vorstehenden Formel (I) in ihrer Gesamtheit für Diphenylamino, Dianisylamino, Morpholinyl, Thiomorpholinyl, 3,5-Dimethylthiomorpholinyl, Piperidinyl, Azacycloheptyl, Azacyclooctyl, 1,4-Diaza-1-methyl-cycloheptyl, Piperazinyl, Pyrrolidinyl und 1,2,3,4-Tetrahydroisochinolinyl.

Wenn die Reste NR₁R₂, R₃ und R₄ zusammen mit dem Benzolring des Naphthopyrangerüsts, an den sie gebunden sind, eine Julolidinyl-Einheit bilden, ergibt sich folgende Struktureinheit:

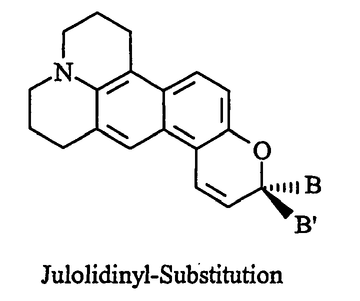

Wenn die Reste R₁ und R₄ bzw. R₂ und R₃ unter Einschließen des Stickstoffatoms jeweils miteinander eine an den Benzolring des Naphthopyrangerüsts gebundene -R₂N-(CH₂)ₖ-X- bzw. -R₁N-(CH₂)ₖ-X- Einheit, wie vorstehend definiert, bilden, so sind folgende Struktureinheiten bevorzugt:

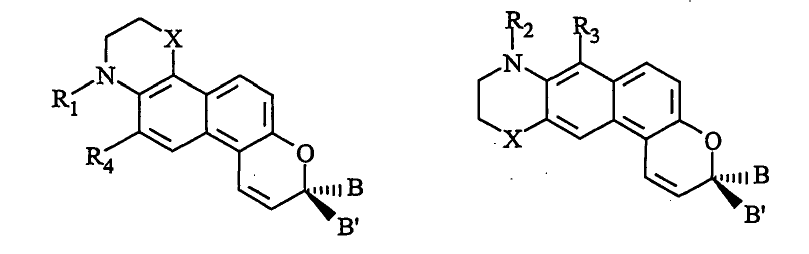

Vorzugsweise ist X in den vorstehenden Struktureinheiten aus O, CH₂ und N(CH₃) ausgewählt.

In einer weiteren bevorzugten Ausführungsform der vorliegenden Erfindung ist der Rest B vorzugsweise aus einem in para-Stellung mit einer -NR₆R₇ Gruppe substituierten Phenylrest ausgewählt, wobei die Reste R₆ und R₇ zusammen mit dem Stickstoffatom der -NR₆R₇ Gruppe eine Azaadamantylgruppe oder einen wie vorstehend definierten 3- bis 10-gliedrigen Stickstoffheterocyclus, insbesondere eine Morpholingruppe, eine Thiomorpholingruppe, eine Piperidingruppe, eine Azacycloheptangruppe, eine Azacyclooctangruppe, eine 1,4-Diaza-1-methylcycloheptangruppe, eine Piperazingruppe, eine N-(N'-(C₁-C₆-Alkyl)piperazingruppe oder eine Pyrrolidingruppe bilden, oder der in para-Stellung mit einer -NR₆R₇ Gruppe substituierte Phenylrest steht im Gesamten für eine in 6-Position gebundene N-Methyl-1,2,3,4-tetrahydrochinolinylgruppe, so daß folgende Struktureinheit vorliegt:

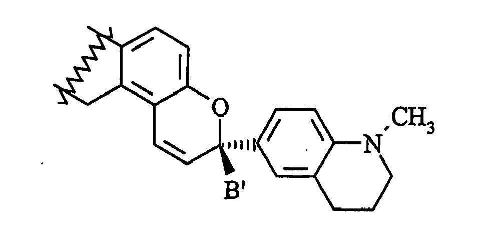

In einer anderen Ausführungsform ist der Rest B vorzugsweise eine 4-Dimethylaminophenylgruppe.

Wenn der Rest B für einen an den Pyranring über die 3-Position gebundenen Julolidinylrest steht, ergibt sich folgende Struktureinheit:

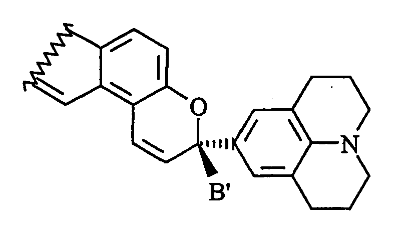

Besonders bevorzugte photochrome Verbindungen 3H-Naphtho[2,1-b]pyrane gemäß der vorliegenden Verbindung sind:
(1) 8-(N-Morpholinyl)-3-phenyl-3-(4-(N-pyrrolidinyl)phenyl)-3H-naphtho[2,1-b]pyran,
(2) 8-(N-Morpholinyl)-3-phenyl-3-(4-(N-piperidinyl)phenyl)-3H-naphtho[2,1-b]pyran,
(3) 3-(4-(N-Azacycloheptyl)phenyl)-8-(N-morpholinyl)-3-phenyl-3H-naphtho-[2,1-b]pyran,
(4) 3-(4-(N-Azacyclooctyl)phenyl)-8-(N-morpholinyl)-3-phenyl-3H-naphtho[2,1-b]pyran,
(5) 3-(4-(N-Azacyclooctyl)phenyl)-8-(N-3,5-dimethylmorpholinyl)-3-phenyl-3H-naphtho[2,1-b]pyran,
(6) 3-(4-(N-Azacyclooctyl)phenyl)-3-phenyl-8-(N-thiomorpholinyl)-3H-naphtho[2,1-b]pyran,
(7) 3-(4-(N-Azacycloheptyl)phenyl)-3-phenyl-8-(N-pyrrolidinyl)-3H-naphtho[2,1-b]pyran,
(8) 3-(4-((Di-4-anisyl)amino)phenyl)-3-phenyl-3-(N-piperidinyl)-3H-naphtho[2,1-b]pyran,
(9) 3-Phenyl-8-(N-piperidinyl)-3-(4-(N-pyrrolidinyl)phenyl)-3H-naphtho[2,1-b]pyran,
(10) 3-(6-(N-Methyl-1,2,3,4-tetrahydrochinolinyl)-3-phenyl-8-(N-piperidinyl)-3H-naphtho[2,1-b]pyran,
(11) 3-(4-(N-Azacycloheptyl)phenyl)-3-phenyl-8-(N-piperidinyl)-3H-naphtho[2,1-b]pyran,
(12) 3-(4-(1,4-Diaza-1-methylcycloheptyl)phenylr8-(N-piperidinyl)-3-phenyl-3H-naphtho[2,1-b]pyran,
(13) 3-(4-(N-Azacyclooctyl)phenyl)-3-phenyl-8-(N-piperidinyl)-3H-naphtho[2,1-b]pyran,
(14) 3-(4-(N-Azacycloheptyl)phenyl)-3-phenyl-8-(N-1,2,3,4-tetrahydroisochinolinyl)-3H-naphtho[2,1-b]pyran,
(15) 3-(4-(N-Morpholinyl)phenyl-3-phenyl-8-(N-1,2,3,4-tetrahydroisochinolinyl)-3H-naphtho[2,1-b]pyran,
(16) 8-(N-Azacycloheptyl)-3-(4-(N-morpholinyl)phenyl)-3-phenyl-3H-naphtho[2,1-b]pyran,
(17) 3-(4(N-Azacycloheptyl)phenyl)-8-diphenylamino-3-phenyl-3H-naphtho[2,1-b]pyran,
(18) 3-((4-Dimethylamino)phenyl)-8-diphenylamino-3-phenyl-3H-naphtho[2,1-b]pyran,
(19) 3-((4-Dimethylamino)phenyl)-8-diphenylamino-3-(2-fluorphenyl)-3H-naphtho[2,1-b]pyran und
(20) 8-Dianisylamino-3-(4-dimethylamino)phenyl-3-phenyl-3H-naphtho[2,1-b]pyran

Die längstwelligen Absorptionsmaxima der offenen Form der vorgenannten beispielhaften photochromen 3H-Naphtho[2,1-b]pyran-Derivate gemäß der vorliegenden Erfindung sind in der nachstehenden Tabelle exemplarisch aufgeführt.

| Verbindung | Längstwelliges Absorptionsmaximum der offenen (farbigen) Form [gemessen in Methacrylatpolymer] | Optischer Farbeindruck |
|---|---|---|
| (1) | 575 nm | Blauviolett |
| (2) | 555 nm | Violett |
| (3) | 580 nm | Blau |
| (4) | 575 nm | Blauviolett |
| (5) | 580 nm | Blau |
| (6) | 585 nm | Blau |
| (7) | 590 nm | Blau |
| (8) | 570 nm | Blauviolett |
| (9) | 580 nm | Blau |
| (10) | 580 nm | Blau |
| (11) | 585 nm | Blau |
| (12) | 570 nm | Violettblau |
| (13) | 580 nm | Blau |
| (14) | 590 nm | Blau |
| (15) | 555 nm | Violett |
| (16) | 580 nm | Blau |
| (17) | 590 nm | Blau |
| (18) | 575 nm | Blauviolett |
| (19) | 575 nm | Blauviolett |
| (20) | 590 nm | Blau |

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen 3H-Naphtho[2,1-b]-pyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzo[f]chromen-Farbstoffe beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen 3H-Naphtho[2,1-b]-pyran-Farbstoffe durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Obertlächenfärbungsverfahren unterschieden. Ein Massefärbungsverfahren umfaßt beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen 3H-Naphtho[2,1-b]-pyran-Farbstoffe beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

Die erfindungsgemäßen photochromen 3H-Naphtho[2,1-b]-pyran-Farbstoffe mit der allgemeinen Formel (I) lassen sich beispielsweise nach dem in Figur 1 gezeigten Reaktionsschema herstellen.

Ausgehend von geeignet substituierten 2-Naphtholen wird zunächst in Schritt i) die Hydroxygruppe geschützt, und zwar vorzugsweise mit einer tert. Butyldiphenylsilylether-Schutzgruppe. Anschließend wird in Schritt ii) mittels Palladiumkatalysierter Aminierung in die 6-Stellung des derart geschützten Naphthols eine Einheit, die eine entsprechende Amingruppe enthält, eingeführt, wobei die Abgangsgruppe in der 6-Stellung des Naphthols ein Brom- oder lodatom oder eine Triflatgrupe sein kann. Nach Abspaltung der Silylether-Schutzgruppe in Schritt iii) werden die derart erhaltenen substituierten 2-Naphthol-Derivate mit geeignet substituierten 2-Propin-1-ol-Derivaten gemäß Schritt iv) zu den erfindungsgemäßen Verbindungen umgesetzt.

## Patentansprüche

1. Photochrome 3H-Naphtho[2,1-b]-pyrane mit der allgemeinen Formel (I): 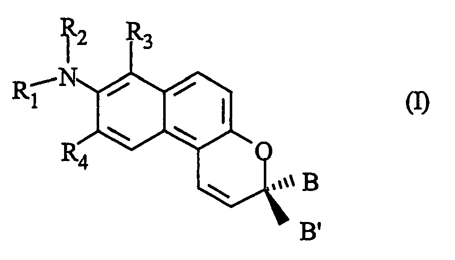 worin
die Reste R₁ und R₂ unabhängig voneinander einen Substituenten darstellen, ausgewählt aus
der Gruppe α, bestehend aus einem geradkettigen oder verzweigtkettigen (C₁-C₆)-Alkylrest, einem (C₃-C₇)-Cycloalkylrest und einem un-, mono- oder disubstituierten Phenyl-, Phenoxy-, Benzyl-, Benzyloxy-, Naphthyl- oder Naphthoxyrest, wobei die Substituenten aus geradkettigen oder verzweigtkettigen (C₁-C₆)-Alkylresten und (C₁-C₆)-Alkoxyresten ausgewählt sein können,
der Gruppe β, worin die Reste R₁ und R₂ zusammen mit dem Stickstoffatom einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁵ enthalten kann, wobei der Rest R⁵ aus einem geradkettigen oder verzweigtkettigen (C₁-C₆)-Alkylrest oder einem unsubstituierten oder mit geradkettigen oder verzweigtkettigen (C₁-C₆)-Alkylresten mono- oder disubstituierten Phenyl- oder Benzylrest ausgewählt sein kann, und wobei der Stickstoffheterocyclus mit einem oder zwei Benzolringen annelliert sein kann, oder
der Gruppe χ, worin die Reste R₁ und R₂ zusammen mit dem Stickstoffatom eine Azaadamantylgruppe bilden;
die Reste R₃ und R₄ unabhängig voneinander aus Wasserstoff, einem geradkettigen oder verzweigtkettigen (C₁-C₆)-A)kylrest, einem (C₃-C₇)-Cycloalkylrest oder einem (C₁-C₆)-Alkoxyrest ausgewählt sind, oder
die Reste R₁ und R₄ bzw. R₂ und R₃ unter Einschließen des Stickstoffatoms jeweils miteinander eine an den Benzolring des Naphthopyrangerüsts gebundene -R₂N-(CH₂)ₖ-X- bzw. -R₁N-(CH₂)ₖ-X- Einheit mit k = 1 oder 2 bilden, wobei X aus O, S, CH₂, C(CH₃)₂, C(C₆H₅)₂, N(CH₃) oder N(C₆H₅) ausgewählt ist und der Rest R₂ bzw. R₁ dann aus Methyl oder Phenyl ausgewählt ist, und wobei an diese -R₂N-(CH₂)ₖ-X- bzw. -R₁N-(CH₂)ₖ-X- Einheit ein Benzolring annelliert sein kann, oder
die Reste NR₁R₂, R₃ und R₄ zusammen mit dem Benzolring des Naphthopyrangerüsts, an den sie gebunden sind, eine Julolidinyl-Einheit bilden,
B ausgewählt ist:
aus einem an den Pyranring über die 3-Position gebundenen Julolidinylrest, einem in ortho- oder para-Stellung mono- oder disubstituierten Phenyl- oder Naphthylrest, wobei der oder die Substituenten eine -NR₆R₇ Gruppe ist bzw. sind, wobei die Reste R₆ und R₇ unabhängig voneinander aus einem (C₃-C₇)-Cycloalkylrest oder einem gegebenenfalls mit einem oder mehreren geradkettigen oder verzweigtkettigen (C₁-C₆)-Alkylresten oder (C₁-C₆)-Alkoxyresten substituierten Phenylrest oder Benzylrest ausgewählt sind, oder die Reste R₆ und R₇ zusammen mit dem Stickstoffatom der -NR₆R₇ Gruppe eine Azaadamantylgruppe oder einen 3- bis 10-gliedrigen Stickstoffheterocyclus bilden, der unsubstituiert oder mit einem geradkettigen oder verzweigtkettigen (C₁-C₆)-Alkylrest substituiert sein kann, wobei der Stickstoffheterocyclus ein oder mehrere Heteroatome aus der Gruppe O, S oder NR⁵ enthalten kann und gegebenenfalls mit einem oder zwei Benzolringen annelliert sein kann, oder zwei direkt benachbarte Substituenten an dem Phenyl- oder Naphthylrest eine an den aromatischen Ring gebundene
-R₈N-(CH₂)ₖ-X- Gruppe mit k = 1 oder 2 bilden, wobei X aus O, S, CH₂, C(CH₃)₂, C(C₆H₅)₂, N(CH₃) oder N(C₆H₅) ausgewählt ist und der Rest R₈ dann aus Methyl oder Phenyl ausgewählt ist, mit der Maßgabe, daß die -R₈N- Einheit in ortho- oder para-Stellung gebunden ist, und
B' aus einem unsubstituierten oder in ortho- oder para-Stellung mono-, di- oder trisubstituierten Phenyl- oder Naphthylrest ausgewählt ist, wobei die Substituenten aus einem geradkettigen oder verzweigtkettigen (C₁-C₆)-Alkylrest, einem (C₃-C₇)-Cycloalkylrest, einem (C₁-C₆)-Alkoxyrest, Fluor, Chlor oder Brom ausgewählt sind, wobei die Phenyl- bzw. Naphthylreste der Einheiten B und B' unabhängig voneinander direkt, über eine Ethylengruppe oder eine Ethindiylgruppe an der 3,3'-Position des 3H-Naphtho[2,1-b]-pyransystems gebunden sein können.

2. 3H-Naphtho[2,1-b]-pyrane nach Anspruch 1, wobei die Reste R₁ und R₂ gemäß der vorstehenden Formel (I) aus einem unsubstituierten, mono- oder disubstituierten Phenyl-, Phenoxy-, Benzyl-, Benzyloxy-, Naphthyl- oder Naphthoxyrest ausgewählt sind oder zusammen mit dem Stickstoffatom einen wie vorstehend definierten 3- bis 10-gliedrigen Stickstoffheterocyclus bilden.

3. 3H-Naphtho[2,1-b]-pyrane nach Anspruch 2, wobei der Stickstoffheterocyclus eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine (N'-(C₁-C₆-Alkyl)piperazingruppe, eine Pyrrolidingruppe, eine Imidazolidingruppe, eine Pyrazolidingruppe, eine Aziridingruppe, eine Azetidingruppe, eine Indolingruppe, eine Carbazolgruppe, eine Phenothiazingruppe, eine Phenazingruppe, eine Phenoxazingruppe, eine Tetrahydrochinolingruppe oder eine Tetrahydroisochinolingruppe ist.

4. 3H-Naphtho[2,1-b]-pyrane nach Anspruch 1, 2 oder 3, wobei die -NR₁R₂ Gruppe in der vorstehenden Formel (I) in ihrer Gesamtheit für Diphenylamino, Dianisylamino, Morpholinyl, Thiomorpholinyl, 3,5-Dimethylthiomorpholinyl, Piperidinyl, Azacycloheptyl, Azacyclooctyl, 1,4-Diaza-1-methyl-cycloheptyl, Piperazinyl, Pyrrolidinyl oder 1,2,3,4-Tetrahydroisochinolinyl steht.

5. 3H-Naphtho[2,1-b]-pyrane nach einem der Ansprüche 1 bis 4, wobei der Rest B aus einem in para-Stellung mit einer -NR₆R₇ Gruppe substituierten Phenylrest ausgewählt ist, wobei die Reste R₆ und R₇ zusammen mit dem Stickstoffatom der -NR₆R₇ Gruppe eine Azaadamantylgruppe oder einen 3- bis 10-gliedrigen Stickstoffheterocyclus, wie vorstehend definiert, bilden.

6. 3H-Naphtho[2,1-b]-pyrane nach Anspruch 5, wobei der Stickstoffheterocyclus eine Morpholingruppe, eine Thiomorpholingruppe, eine Piperidingruppe, eine Azacycloheptangruppe, eine Azacyclooctangruppe, eine 1,4-Diaza-1-methyl-cycloheptangruppe, eine Piperazingruppe, eine (N'-(C₁-C₆-Alkyl)piperazingruppe oder eine Pyrrolidingruppe ist oder der in para-Stellung mit einer -NR₆R₇ Gruppe substituierte Phenylrest im Gesamten für eine in 6-Position gebundene N-Methyltetrahydrochinolinylgruppe steht.

7. 3H-Naphtho[2,1-b]-pyrane nach einem der vorhergehenden Ansprüche, welche
8-(N-Morpholinyl)-3-phenyl-3-(4-(N-pyrrolidinyl)phenyl)-3H-naphtho[2,1-b]pyran,
8-(N-Morpholinyl)-3-phenyl-3-(4-(N-piperidinyl)phenyl)-3H-naphtho[2,1-b]pyran,
3-(4-(N-Azacycloheptyl)phenyl)-8-(N-morpholinyl)-3-phenyl-3H-naphtho-[2,1-b]pyran,
3-(4-(N-Azacyclooctyl)phenyl)-8-(N-morpholinyl)-3-phenyl-3H-naphtho[2,1-b]pyran,
3-(4-(N-Azacyclooctyl)phenyl)-8-(N-3,5-dimethylmorpholinyl}-3-phenyl-3H-naphtho[2,1-b]pyran,
3-(4-(N-Azacyclooctyl)phenyl)-3-phenyl-8-(N-thiomorpholinyl)-3H-naphtho[2,1-b]pyran,
3-(4-(N-Azacycloheptyl)phenyl)-3-phenyl-8-(N-pyrrolidinyl)-3H-naphtho[2,1 - b]pyran,
3-(4-((Di-4-anisyl)amino)phenyl)-3-phenyl-8-(N-piperidinyl)-3H-naphtho[2,1-b]pyran,
3-Phenyl-8-(N-piperidinyl)-3-(4-(N-pyrrolidinyl)phenyil)-3H-naphtho[2,1-b]pyran,
3-(6-(N-Methyl-1,2,3,4-tetrahydrochinolinyl)-3-phenyl-8-(N-piperidinyl)-3H-naphtho[2,1-b]pyran,
3-(4-(N-Azacycloheptyl)phenyl)-3-phenyl-8-(N-piperidinyl)-3H-naphtho[2,1-b]pyran,
3-(4-(1,4-Diaza-1-methylcycloheptyl)phenyl)-8-(N-piperidinyl)-3-phenyl-3H-naphtho[2,1-b]pyran,
3-(4-(N-Azacyclooctyl)phenyl)-3-phenyl-8-(N-piperidinyl)-3H-naphtho[2,1-b]pyran,
3-(4-(N-Azacycloheptyl)phenyl)-3-phenyl-8-(N-1,2,3,4-tetrahydroisochino-linyl)-3H-naphtho[2,1-b]pyran,
3-(4-(N-Morpholinyl)phenyl-3-phenyl-8-(N-1,2,3,4-tetrahydroisochinolinyl)-3H-naphtho[2,1-b]pyran,
8-(N-Azacycloheptyl)-3-(4-(N-morpholinyl)phenyl)-3-phenyl-3H-naphtho[2,1-b]pyran,
3-(4(N-Azacycloheptyl)phenyl)-8-diphenylamino-3-phenyl)-3H-naphtho[2,1-b]pyran,
3-((4-Dimethylamino)phenyl)-8-diphenylamino-3-phenyl-3H-naphtho[2,1-b]pyran,
3-((4-Dimethylamino)phenyl)-8-diphenylamino-3-(2-fluorphenyl)-3H-naphtho[2,1-b]pyran und
8-Dianisylamino-3-(4-dimethylamino)phenyl-3-phenyl-3H-naphtho[2,1-b]pyran sind.

8. Verwendung der photochromen 3H-Naphtho[2,1-b]-pyrane nach einem der Ansprüche 1 bis 7 in Kunststoffmaterialien.

9. Verwendung nach Anspruch 8, wobei das Kunststoffmaterial eine ophthalmische Linse ist.

## Claims

1. Photochromic 3H-naphtho[2,1-b]-pyrans of the general formula (I): 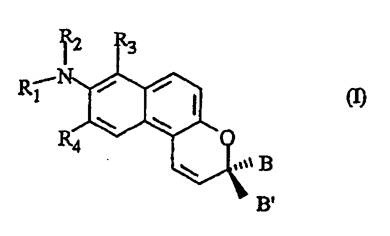 in which
the radicals R₁ and R₂, independently of one another, represent a substituent selected from:
the group α, consisting of a linear or branched (C₁-C₆) alkyl radical, a (C₃-C₇) cycloalkyl radical, and an unsubstituted, monosubstituted or disubstituted phenyl, phenoxy, benzyl, benzyloxy, naphthyl or naphthoxy radical, wherein the substituents can be selected from linear or branched (C₁-C₆) alkyl radicals and (C₁-C₆) alkoxy radicals,
the group β**,** in which the radicals R₁ and R₂ together with the nitrogen atom form a 3 to 10-membered nitrogen-containing heterocyclic compound, which may be unsubstituted or substituted with a linear or branched (C₁-C₆) alkyl radical, wherein the nitrogen-containing heterocyclic compound contains one or more heteroatoms from the group of O, S or NR , wherein the radical R⁵ can be selected from a linear or branched (C₁-C₆) alkyl radical, or a phenyl or benzyl radical, unsubstituted or monosubstituted or disubstituted with linear or branched (C₁-C₆) alkyl radicals, and wherein the nitrogen-containing heterocyclic compound can be annelated with one or two benzene rings, or
the group χ, in which the radicals R₁ and R₂ together with the nitrogen atom form an azaadamantyl group,
the radicals R₃ and R₄, independently of one another, are selected from hydrogen, a linear or branched (C₁-C₆) alkyl radical, a (C₃-C₇) cycloalkyl radical, or a (C₁-C₆) alkoxy radical, or
the radicals R₁ and R₄ or R₂ and R₃ together with the nitrogen atom respectively form with each other an -R₂N-(CH₂)ₖ-X- or -R₁N-(CH₂)k-X- unit, where k = 1 or 2 and which is bound to the benzene ring of the naphthopyran skeleton, wherein X is selected from O, S, CH₂, C(CH₃)₂, C(C₆H₅)₂, N(CH₃) or N(C₆H₅), and the radical R₂ or R₁ is then selected from methyl or phenyl, and wherein a benzene ring can be annelated to this -R₂N-(CH₂)ₖ-X- or -R₁N-(CH₂)ₖ-X- unit, or
the radicals NR₁R₂, R₃ and R₄ together with the benzene ring of the naphthopyran skeleton to which they are bound form a julolidinyl unit,
B is selected:
from a julolidinyl radical bound via the 3 position to the pyran ring, a phenyl or naphthyl radical, monosubstituted or disubstituted in the ortho or para position, wherein the substituent or substituents is or are a -NR₆R₇ group, wherein the radicals R₆ and R₇ independently of one another are selected from a (C₃-C₇) cycloalkyl radical or a phenyl or benzyl radical, optionally substituted by one or more linear or branched (C₁-C₆) alkyl radicals or (C₁-C₆) alkoxy radicals, or the radicals R₆ and R₇, together with the nitrogen atom of the -NR₆R₇ group form an azaadamantyl group or a 3 to 10-membered nitrogen-containing heterocyclic compound, which may be unsubstituted or substituted by a linear or branched (C₁-C₆) alkyl radical, wherein said nitrogen-containing heterocyclic compound can contain one or more heteroatoms selected from the group of O, S or NR⁵ and can optionally be annelated with one or two benzene rings, or two directly adjacent substituents on said phenyl or naphthyl radical form an -R₈N-(CH₂)ₖ-X group bound to the aromatic ring, where k = 1 or 2, wherein X is selected from O, S, CH₂, C(CH₃)₂, C(C₆H₅)₂, N(CH₃) or N(C₆H₅), and the radical R₈ is then selected from methyl or phenyl, with the proviso that the -R₈N unit is bound in the ortho or para position, and
B' is selected from a phenyl or naphthyl radical, which is unsubstituted, or monosubstituted, disubstituted or trisubstituted in the ortho or para position, wherein the substituents are selected from a linear or branched (C₁-C₆) alkyl radical, a (C₃-C₇) cycloalkyl radical, a (C₁-C₆) alkoxy radical, fluorine, chlorine or bromine, wherein the phenyl or naphthyl radicals of the units B and B' independently of one another can be bound directly, via an ethylene group or an ethinediyl group, at the 3,3'-position of the 3H-naphtho[2,1-b]-pyran system.

2. 3H-naphtho[2,1-b]-pyrans according to claim 1, wherein the radicals R₁ and R₂, according to formula (1) above, are selected from an unsubstituted, monosubstituted or disubstituted phenyl, phenoxy, benzyl, benzyloxy, naphthyl or naphthoxy radical, or together with the nitrogen atom form a 3 to 10-membered nitrogen-containing heterocyclic compound, as defined hereinbefore.

3. 3H-naphtho[2,1-b]-pyrans according to claim 2, wherein the nitrogen-containing heterocyclic compound is a morpholine group, a thiomorpholine group, a piperidine group, an azacycloheptane group, an azacyclooctane group, a 1,4-diaza-l-methyl-cycloheptane group, a piperazine group, an (N'-(C₁-C₆-alkyl)piperazine group, a pyrrolidine group, an imidazolidine group, a pyrazolidine group, an aziridine group, an azetidine group, an indoline group, a carbazole group, a phenothiazine group, a phenazine group, a phenoxazine group, a tetrahydroquinoline group, or a tetrahydrosioquinoline group.

4. 3H-naphtho[2,1-b]-pyrans according to any one of claims 1, 2 or 3, wherein the -NR₁R₂ group in formula (I) above represents in its entirety a diphenylamino, dianisylamino, morpholinyl, thiomorpholinyl, 3,5-dimethylthiomorpholinyl, piperidinyl, azacycloheptyl, azacyclooctyl, 1,4-diaza-1-methyl-cycloheptyl, piperazinyl, pyrrolidinyl or 1,2,3,4-tetrahydroisoquinolinyl group.

5. 3H-naphtho[2,1-b]-pyrans according to any one of claims 1 to 4, wherein the radical B is selected from a phenyl radical substituted in the para position by an -NR₆R₇ group, wherein the radicals R₆ and R₇, together with the nitrogen atom of the -NR₆R₇ group, form an azaadamantyl group or a 3 to 10-membered nitrogen-containing heterocyclic compound, as defined hereinbefore.

6. 3H-naphtho[2,1-b]-pyrans according to claim 5, wherein the nitrogen-containing heterocyclic compound is a morpholine group, a thiomorpholine group, a piperidine group, an azacycloheptane group, an azacyclooctane group, a 1,4-diaza-1-methylcycloheptane group, a piperazine group, an (N'-(C₁-C₆ alkyl)piperazine group or a pyrrolidine group, or the phenyl radical substituted in the para position by a -NR₆R₇ group represents in its entirety an N-methyltetrahydroquinoline group bound in the 6-position.

7. 3H-naphtho[2,1-b]-pyrans according to any one of the preceding claims, which are:
8-(N-morpholinyl)-3-phenyl-3-(4-(N-pyrrolidinyl)phenyl)-3H-naphtho[2,1-b]pyran,
8-(N-morpholinyl)-3-phenyl-3-(4-(N-piperidinyl)phenyl)-3H-naphtho[2,1-b]pyran,
3-(4-(N-azacycloheptyl)phenyl)-8-(N-morpholinyl)-3-phenyl-3H-naphtho-[2,1-b]pyran,
3-(4-(N-azacyclooctyl)phenyl)-8-(N-morpholinyl)-3-phenyl-3H-naphtho-[2,1-b]pyran,
3-(4-(N-azacyclooctyl)phenyl)-8-(N-3,5-dimethylmorpholinyl)-3-phenyl-3H-naphtho-[2,1-b] pyran,
3-(4-(N-azacyclooctyl)phenyl)-3-phenyl-8-(N-thiomorpholinyl)-3H-naphtho-[2,1-b]pyran,
3-(4-(N-azacycloheptyl)phenyl)-3-phenyl-8-(N-pyrrolidinyl)-3H-naphtho-[2,1-b]pyran,
3-(4-((di-4-anisyl)amino)phenyl)-3-phenyl-8-(N-piperidinyl)-3H-naphtho-[2,1-b]pyran,
3-phenyl-8-(N-piperidinyl)-3-(4-(N-pyrrolidinyl)phenyl)-3H-naphtho-[2,1-b]pyran,
3-(6-(N-methyl-1,2,3,4-tetrahydroquinolinyl)-3-phenyl-8-(N-piperidinyl)-3H-naphtho-[2,1-b] pyran,
3-(4-(N-azacyclooctyl)phenyl)-3-phenyl-8-(N-piperidinyl)-3H-naphtho-[2,1-b]pyran,
3-(4-(1,4-diaza-1-methylcycloheptyl)phenyl)-8-(N-piperidinyl)-3-phenyl-3H-naphtho-[2,1-b]pyran,
3-(4-(N-azacyclooctyl)phenyl)-3-phenyl-8-(N-piperidinyl)-3H-naphtho-[2,1-b]pyran,
3-(4-(N-azacycloheptyl)phenyl)-3-phenyl-8-(N-1,2,3,4-tetrahydroisoquinolinyl)-3H-naphtho-[2,1-b]pyran,
3-(4-(N-morpholinyl)phenyl)-3-phenyl-8-(N-1,2,3,4-tetrahydroisoquinolinyl)-3H-naphtho-[2,1-b]pyran,
8-(N-azacycloheptyl)-3-(4-(N-morpholinyl)phenyl)-3-phenyl-3H-naphtho-[2,1-b]pyran,
3-(4-(N-azacycloheptyl)phenyl)-8-diphenylamino-3-phenyl-3H-naphtho-[2,1-b]pyran,
3-((4-dimethylamino)phenyl)-8-diphenylamino-3-phenyl-3H-naphtho-[2,1-b]pyran,
3-((4-dimethylamino)phenyl)-8-diphenylamino-3-(2-fluorophenyl)-3H-naphtho-[2,1-b]pyran, and
8-dianisylamino-3-(4-dimethylamino)phenyl-3-phenyl-3H-naphtho-[2,1-b]pyran.

8. Use of the photocromic 3H-naphtho[2,1-b]-pyrans according to any one of claims 1 to 7 in plastics materials.

9. Use according to claim 8, wherein the plastics material is an ophthalmic lens.

## Revendications

1. 3H-naphto[2,1-b]-pyranes photochromes de formule générale (1) : 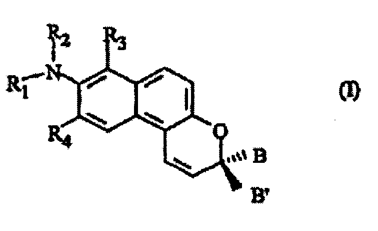 dans laquelle
les radicaux R₁ et R₂ représentent, indépendamment les uns des autres, un substituant choisi parmi
le groupe α, composé d'un radical alkyle en C₁-C₆ à chaîne droite ou à chaîne ramifiée, un radical cycloalkyle en C₃-C₇ et un radical phényle, phénoxy, benzyle, benzyloxy, naphtyle ou naphtoxy non substitué, monosubstitué ou disubstitué, les substituants pouvant être choisis parmi des radicaux alkyles en C₁-C₆ et des radicaux alcoxy en C₁-C₆ à chaîne droite ou à chaîne ramifiée,
le groupe β, dans lequel les radicaux R₁ et R₂ forment avec l'atome d'azote un hétérocycle d'azote de 3 à 10 chaînons, pouvant être non substitué ou substitué avec un radical alkyle en C₁-C₆ à chaîne droite ou à chaîne ramifiée, l'hétérocycle d'azote pouvant contenir un ou plusieurs hétéroatomes du groupe O, S ou NR₅, le radical R⁵ pouvant être choisi parmi un radical alkyle en C₁-C₆ à chaîne droite ou à chaîne ramifiée ou un radical benzyle ou phényle non substitué ou monosubstitué ou disubstitué avec des radicaux alkyles en C₁-C₆ à chaîne droite ou à chaîne ramifiée, et l'hétérocycle d'azote pouvant être annelé avec un ou deux noyaux benzéniques, ou
le groupe X, dans lequel les radicaux R₁ et R₂ forment avec l'atome d'azote un groupe azaadamantyle ;
les radicaux R₃ et R₄ sont choisis, indépendamment les uns des autres, parmi de l'hydrogène, un radical alkyle en C₁-C₆ à chaîne droite ou à chaîne ramifiée, un radical cycloalkyle en C₃-C₇ ou un radical alcoxy en C₁-C₆, ou
les radicaux R₁ et R₄ ou R₂ et R₃ forment respectivement ensemble, en incluant l'atome d'azote, une unité -R₂N-(CH₂)ₖ-X- ou -R₁N-(CH₂)ₖ-X- liée au noyau benzénique du squelette de naphtopyrane avec k = 1 ou 2, X étant choisi parmi O, S, CH₂, C(CH₃)₂, C(C₆H₅)₂, N(CH₃) ou N(C₆H₅) et le radical R₂ et/ou R₁ étant alors choisi parmi le méthyle ou le phényle et un anneau benzénique pouvant être annelé à cette unité -R₂N-(CH₂)ₖ-X- ou -R₁N-(CH₂)ₖ-X-; ou
les radicaux NR₁R₂, R₃ et R₄ formant avec l'anneau benzénique du squelette de naphtopyrane auquel ils sont liés, une unité julolidinyle,
B est choisi parmi:
un radical julolidinyle lié à un noyau pyranique par la position 3, un radical phényle ou naphtyle monosubstitué ou disubstitué en position para ou ortho, le ou les substituants étant un groupe -NR₆R₇, les radicaux R₆ et R₇ étant choisis, indépendamment les uns des autres, parmi un radical cycloalkyle en C₃-C₇ ou un radical phényle ou benzyle substitué, le cas échéant, avec un ou plusieurs radicaux alcoxy en C₁-C₆ ou radicaux alkyles en C₁-C₆ à chaîne droite ou à chaîne ramifiée, ou les radicaux R₆ et R₇ formant, avec l'atome d'azote du groupe -NR₆R₇, un groupe azaadamantyle ou un hétérocycle d' azote de 3 à 10 chaînons pouvant être non substitué ou substitué avec un radical alkyle en C₁-C₆ à chaîne droite ou à chaîne ramifiée, l'hétérocycle d'azote pouvant contenir un ou plusieurs hétéroatomes du groupe O, S ou NR⁵ et pouvant être annelé, le cas échéant, avec un ou deux noyaux benzéniques, ou deux substituants directement voisins formant, au niveau du radical phényle ou naphtyle, un groupe -R₈N-(CH₂)ₖ-X- lié au noyau aromatique avec k = 1 ou 2, X étant choisi parmi O, S, CH₂, C(CH₃)₂, C(C₆H₅)₂, N(CH₃) ou N(C₆H₅), et le radical R₈ étant alors choisi parmi le méthyle ou le phényle, dans la mesure où l'unité -R₈N- soit liée en position ortho ou para, et
B' est choisi parmi un radical phényle ou naphtyle non substitué ou monosubstitué, disubstitué ou trisubstitué en position ortho ou para, les substituants étant choisis parmi un radical alkyle en C₁-C₆ à chaîne droite ou à chaîne ramifiée, un radical cycloalkyle en C₃-C₇,- un radical alcoxy en C₁-C₆, du fluor, du chlore ou du brome, les radicaux phényle ou naphtyle des unités B et B' pouvant être liés, indépendamment les uns des autres, directement, par le biais d'un groupe éthylène ou d'un groupe éthindiyle, à la position 3,3' du système 3H-naphto[2,1-b]-pyrane.

2. 3H-naphto[2,1-b]-pyranes selon la revendication 1, les radicaux R₁ et R₂ selon la formule (I) précédente, étant choisis parmi un radical phényle, phénoxy, benzyle, benzyloxy, naphtyle, ou naphtoxy non substitué, monosubstitué ou disubstitué, ou formant avec l'atome d'azote, un hétérocyle d'azote de 3 à 10 chaînons comme défini précédemment.

3. 3H-naphto[2,1-b]-pyrane selon la revendication 2, l'hétérocycle d'azote étant choisi parmi un groupe morpholine, un groupe thiomorpholine, un groupe pipéridine, un groupe azacycloheptane, un groupe azacyclooctane, un groupe 1,4-diaza-1-méthyl-cycloheptane, un groupe pipérazine, un groupe (N'-(C₁-C₆-alkyle)pipérazine, un groupe pyrrolidine, un groupe imidazolidine, un groupe pyrazolidine, un groupe aziridine, un groupe azétidine, un groupe indoline, un groupe carbazole, un groupe phénothiazine, un groupe phénazine, un groupe phénoxazine, un groupe tétrahydrochinoline ou un groupe tétrahydroisochinoline.

4. 3H-naphto[2,1-b]-pyranes selon la revendication 1, 2 ou 3, le groupe -NR₁R₂ dans la formule (I) précédente représentant, dans sa totalité, diphénylamino, dianisylamino, morpholinyle, thiomorpholinyle, 3,5-diméthylthiomorpholinyle, pipéridinyle, azacycloheptyle, azacyclooctyle, 1,4-diaza-1-méthyl-cycloheptyle, pipérazinyle, pyrrolidinyle ou 1,2,3,4-tétrahydroisochinolinyle.

5. 3H-naphto[2,1-b]-pyranes selon l'une quelconque des revendications 1 à 4, le radical B étant choisi parmi un radical phényle substitué avec un groupe -NR₆R₇ en position para, les radicaux R₆ et R₇ formant, avec l'atome d'azote du groupe -NR₆R₇, un groupe azaadamantyle ou un hétérocycle d'azote de 3 à 10 chaînons, comme défini précédemment.

6. 3H-naphto[2,1-b]-pyranes selon la revendication 5, l'hétérocycle d'azote étant un groupe morpholine, un groupe thiomorpholine, un groupe pipéridine, un groupe azacycloheptane, un groupe azacyclooctane, un groupe 1,4-diaza-1-méthyl-cycloheptane, un groupe pipérazine, un groupe (N'- (C₁-C₆-alkyle) pipérazine ou un groupe pyrrolidine ou le radical phényle substitué avec un groupe -NR₆R₇ en position para représente, dans sa totalité, un groupe N-méthyltétrahydrochinolinyle lié en position 6.

7. 3H-naphto[2,1-b]-pyranes selon l'une quelconque des revendications précédentes qui sont
8-(N-morpholinyl)-3-phényl-3-(4-(N-pyrrolidinyl)phényl)-3H-naphto[2,1-b]pyrane,
8-(N-morpholinyl)-3-phényl-3-(4-(N-pipéridinyl)phényl)-3H-naphto[2,1-b]pyrane,
3-(4-(N-azacycloheptyl)phényl)-8-(N-moprholinyl)-3-phényl-3H-naphto-[2,1-b]pyrane,
3-(4-(N-azacyclooctyl)phényl)-8-(N-moprholinyl)-3-phényl-3H-naphto-[2,1-b]pyrane,
3- (4- (N-azacyclooctyl) phényl) -8- (N-3, 5-diméthylmorpholinyl)-3-phényl-3H-naphto-[2,1-b]pyrane,
3-(4-(N-azacyclooctyl)phényl)-3-phényl-8-(N-thiomorpholinyl)-3H-naphto-[2,1-b]pyrane,
3-(4-(N-azacycloheptyl)phényl)-3-phényl-8-(N-pyrrolidinyl)-3H-naphto-[2,1-b]pyrane,
3-(4-((Di-4-anisyl)amino)phényl)-3-phényl-8-(N-pipéridinyl)-3H-naphto-[2,1-b]pyrane,
3-phényl-8-(N-pipéridinyl)-3-(4-(N-pyrrolidinyl)phényl)-3H-naphto[2,1-b]pyrane,
3-(6-(N-méthyl-1,2,3,4-tétrahydrochinolinyl)-3-phényl-8-(N-pipéridinyl)-3H-naphto[2,1-b]pyrane,
3-(4-(N-azacycloheptyl)phényl)-3-phényl-8-(N-pipéridinyl)-3H-naphto[2,1-b]pyrane,
3-(4-(1,4-diaza-1-méthycycloheptyl)phényl)-8-(N-pipéridinyl)-3-phényl-3H-naphto[2,1-b]pyrane,
3-(4-(N-azacyclooctyl)phényl)-3-phényl-8-(N-pipéridinyl)-3H-naphto[2,1-b]pyrane,
3-(4-(N-azacycloheptyl)phényl)-3-phényl-8-(N-1,2,3,4-tétrahydroisochinolinyl)-3H-naphto[2,1-b]pyrane,
3-(4-(N-morpholinyl)phényl)-3-phényl-8-(N-1,2,3,4-tétrahydroisochinolinyl)-3H-naphto[2,1-b]pyrane,
8-(N-azacycloheptyl)-3-(4-(N-morpholinyl)phényl)-3-phényl-3H-naphto[2,1-b]pyrane,
3-(4(N-azacycloheptyl)phényl)-8-diphénylamino-3-phényl-3H-naphto[2,1-b]pyrane,
3-((4-diméthylamino)phényl)-8-diphénylamino-3-phényl-3H-naphto[2,1-b]pyrane,
3-((4-diméthylamino)phényl)-8-diphénylamino-3-(2-fluorophényl)-3H-naphto[2,1-b]pyrane, et
8-dianisylamino-3-(4-diméthylamino)phényl-3-phényl-3H-naphto[2,1-b]pyrane.

8. Utilisation des 3H-naphto[2,1-b]pyranes photochromes selon l'une quelconque des revendications 1 à 7 dans des matières plastiques.

9. Utilisation selon la revendication 8, la matière plastique étant une lentille ophtalmique.
